# EUROPEAN PATENT APPLICATION

(11) **EP 2 324 812 A2**
(43) Date of publication of application: **25.05.2011**
(21) Application number: 10182378.9
(22) Date of filing: 20.04.2001
(51) Int. Cl.: A61K 8/11

(54) **Composition exhibiting enhanced formulation stability and delivery of topical active ingredients**

(30) Priority: 21.04.2000 US 198749 P
(62) Divisional of application: 01925838.3
(71) Applicant: Sol-Gel Technologies Ltd., Beit Shemesh 99100 (IL)
(72) Inventor: Lapidot, Noa, 90805, Mevasseret Zion (IL); Magdassi, Shlomo, 96626, Jerusalem (IL); Avnir, David, 96908, Jerusalem (IL); Rottman, Claudio, 97830, Jerusalem (IL); Ben Zazon, Orit, Ein Tsurim 79510 (IL); Seri-Levy, Alon, 76248, Rehovot (IL)
(74) Representative: Fichter, Robert Arno

(57) **Abstract**

The present invention concerns a process for the preparation of microcapsules having a core-shell structure, wherein said microcapsules have a diameter of approximately 0.1 to 100 micron, wherein each core includes at least one active ingredient, said core is encapsulated within a microcapsular shell, and wherein said shell is comprised of at least one inorganic polymer obtained by a sol-gel process, and said microcapsular shell protects the active ingredient prior to topical application and releases said active ingredient after topical application, said process comprises the steps of; (a) preparing a hydrophobic solution or a hydrophobic dispersion comprising sol-gel precursors and an ingredient to be encapsulated; (b) emulsifying the hydrophobic solution or dispersion of step (a) in an aqueous solution under high shear forces to obtain an emulsion; (c) mixing and stirring the emulsion obtained, with a second aqueous solution, at a selected pH to obtain the sol-gel microcapsules.

## Description

### FIELD OF THE INVENTION

The present invention relates to a delivery system for topically applied therapeutic or cosmetic ingredients, based on encapsulation of the active ingredients in sol-gel microcapsules. More particularly, the microcapsules of the present invention, serve to protect ingredients that decompose when in contact with oxygen, water or with other ingredients of the topical formulation, by acting as a protecting barrier, thus enhancing the stability of these sensitive ingredients in the formulation. The microcapsules are additionally useful to limit contact of skin-irritant active ingredients. The capsules are also capable of releasing the encapsulated ingredients once applied through a pre-designed release mechanism, thus acting as a delivery system.

The present invention further relates to a process for production of said microcapsules.

### BACKGROUND OF THE INVENTION

Many active ingredients which are recognized as beneficial for improving skin or hair condition and feeling, for reducing signs of aging and photoaging, or for the treatment of skin disorders such as acne, psoriasis, seborrhea and infections, are difficult to formulate in cosmetic compositions or in pharmaceutical preparations. Often these active ingredients decompose when in contact with water, oxygen, oxidants, trace amounts of metallic ions, or with components commonly used in cosmetic or dermatological compositions. Consequently, the shelf life of products containing these ingredients is reduced.

Another prevalent problem is that while being effective in treating the skin, many of these active ingredients cause skin irritation. A delivery system for sustained release can contribute to decreasing such irritation by reducing the concentration of active ingredients, that are in contact with the skin or hair, at any given moment. Sustained release can extend the duration of activity of the ingredient.

Hence, a delivery system that is capable of holding and protecting the sensitive active ingredient in the formulation, and of delivering the active ingredient upon application is advantageous. An effective delivery system should stabilize the sensitive active ingredient against undesired decomposition, thus extending the shelf life of the composition. The encapsulation may also serve to separate and segregate incompatible agents present in the same composition. The activity of the ingredients can be prolonged as a result of the sustained release, while skin irritation can be reduced, since there is a significant reduction in the concentration of active ingredient in direct contact with the skin, at any given moment.

Perfumes are an example of an ingredient that is frequently added to the composition. Perfumes, while having no therapeutic action, often cause skin irritation. Entrapment of perfumes may serve to decrease skin sensitivity to perfumes, while extending their period of effectiveness through the mechanism of sustained release. Colors and dyes can also benefit from entrapment, since they are often incompatible with other formulation ingredients.

Various formulations have been developed to address these problems. Improved emulsions of w/o or o/w have been developed, such as that described in U.S. Patent No. 6,171,600, which discloses use of a double emulsion. U.S. Patent No. 5,851,538 discloses a protection system based on the adsorption of the active ingredient in pores that are present in an organic polymer in a sponge form. US Patent No. 3,957,971, and US Patent No. 5,874,105 utilize liposomes as a delivery system.

U.S Patent No. 6,103,267 and U.S Patent No. 6,146,664 show that sensitive active ingredients, such as Vitamin A and Vitamin C, can be stabilized as dispersions in a non-solvent, and still be active when applied on the skin. A similar approach was used in U.S. Patent No. 6,077,522, for stabilizing biologically active compounds for various uses.

Another media for controlled delivery of drugs, which can be utilized to protect sensitive ingredients, is doping within sol-gel matrices. In this method, monoliths, particles or other forms (such as thin layers, or fibers) are made, and the active ingredient is immobilized in the pores of the sol-gel matrix. The sol-gel matrix is doped with small amounts of the active ingredient. This method is utilized in U.S. Patent No. 5,591,453, WO 9745367, U.S. Patent No. 4,169,069, DE 19811900, WO 00/47236 and US 4,988,744. Sol-gel doped matrices cannot support high loading (of up to 95% wt.) of the active ingredient. In order to obtain high loading, it is essential to form a core-shell structure, where most of the weight of the capsule is the weight of the encapsulated active ingredient, and where the thin shell protects the core effectively.

U.S. Patent Application No. 09/372,176 discloses a method for the preparation of silica microcapsules containing organic compounds for various uses. This method was utilized in the development of encapsulated sunscreen active ingredients, disclosed in U.S. Patent Application No. 09/318,828; where active ingredients are highly retained within the silica capsules, minimalizing exposure of the skin to the active ingredient. Sol-gel microcapsules of silica, when formed as in U.S. Patent Application 09/372,176, are chemically and photochemically stable, inert and safe for use. When incorporated in cosmetic or pharmaceutical compositions they affords a transparent, cosmetically pleasing product. The hydrophobic/hydrophilic character of the capsules is tailored to suit the purpose, by selecting appropriate sol-gel precursors and reaction conditions. Selection of the makeup of the microcapsule precursors, determines the character of the microcapsular shell surrounding the active ingredient. Thus, for instance, hydophobicity/hydrophilicity can be controlled, so that water-soluble actives and oil-soluble actives can both be present in the same formulation, by encapsulation of one or the other. It is possible to encapsulate hydrophobic materials, that would have required the presence of large quantities of oils in the formulation, in silica, which has a hydrophilic external surface, allowing easy incorporation into aqueous phases. Generally, water based products or emulsions with external water phase are considered to afford improved feel on the skin, and are therefore preferred in many cases over oil-based products (ointments) or water in oil emulsions. For that reason it is desirable to have a delivery system that is water dispersible, to allow easy incorporation in the water phase.

Surprisingly, the applicants have discovered that sol-gel microcapsules can be used for the purpose of topical delivery of sensitive active ingredients. U.S. Patent Applications 09/318,828, 09/372,176 (incorporated herein by reference) disclose microcapsule formulations that prevent an encapsulated active ingredient from leaving the microcapsule. This is desirable when the active ingredient is either unstable when in contact with other ingredients in the formulation or with the environment, or when the active ingredient is an irritant to the body tissue to which it is applied. The applicants of the present invention, have discovered that sol-gel microcapsules can be designed to achieve triggered release of their content upon application in a sustained or an immediate manner. The capsules formed in a sol-gel process in the present invention, protect a sensitive ingredient prior to topical administration, increasing its stability and extending its shelf life. The sol-gel capsules allows stabilization of the active ingredient within a cosmetic formulation for a prolonged period of time, by creating a micro-domain or a protective layer around the sensitive ingredient. After topical application of the compositions of the present invention, the microcapsules break and release their contents, thus, they function as a delivery system. While conventional microcapsules are prepared by coating the core material with organic polymers, in the present invention the core material is coated with inorganic polymers. This imparts unique properties to the microcapsule wall, such as rigidity, and sensitivity to friction, which result in breakage of the capsules and release of their contents, during or after application to the skin. The use of inorganic polymers for the microcapsular wall further grants the ability to control the pore size of the microcapsular shell, and eliminates sensitivity of the shell to both organic solvents in the formulation, and to components of the skin.

Additional aspects of the present invention will become more apparent from the detailed description of the preferred embodiments, that follows below.

In the present invention, the term "active ingredient" refers to an ingredient having a therapeutical or cosmetic activity.

In the present invention, the term "topical application" refers to an application on the skin, hair, ears, mucous membranes, rectal application, nasal application, as well as dental application within the oral cavity.

In the present invention, the term "adjuvant" refers to a material used in conjunction with the active ingredient to preserve the stability of the active ingredient within the composition.

In the present invention, the term "BHT" refers to butylated hydroxy toluene.

In the present invention, the term "BHA" refers to butylated hydroxy anisole.

In the present invention, the term "vitamin" refers to any acceptable vitamin, a derivative thereof and a salt thereof.

In the present invention, the term "dental agent" refers to a tooth whitener, a cleanser, a flavor for a toothpaste or mouthwash, a vitamin or other substance having a therapeutic effect on the teeth or oral cavity.

In the present invention, the term "TEOS" refers to tetraethoxy silane, which is a precursor of silica.

### SUMMARY OF THE INVENTION

There is thus provided in accordance with a preferred embodiment of the present invention, a therapeutic or cosmetic composition for topical application, capable of stabilizing an active ingredient and delivering said ingredient. The composition is comprised of a plurality of microcapsules having a core-shell structure, wherein said microcapsules have a diameter of approximately 0.1 to 100 micron. Each core includes at least one active ingredient, and said core is encapsulated within a microcapsular shell. The shell is comprised of at least one inorganic polymer obtained by a sol-gel process, and said microcapsular shell protects the active ingredient prior to topical application and releases said active ingredient after topical application.

Further in accordance with a preferred embodiment of the present invention, the composition comprises:
(a) a plurality of microcapsules encapsulating at least one active ingredient;
(b) a plurality of microcapsules encapsulating an active ingredient, wherein at least one of said active ingredient is different than at least one of the active ingredient in (a).

Moreover, in accordance with a preferred embodiment of the present invention, the composition further comprising a pharmaceutical or a cosmetic carrier. In certain embodiments, the carrier comprises at least one non-encapsulated active ingredient. In other embodiment, the carrier is selected from the group consisting of an emulsion, a cream, an aqueous solution, an oil, an ointment, a paste, a gel, a lotion, a milk, a suspension, or a powder.

Additionally, in accordance with a preferred embodiment of the present invention, the carrier comprises at least one member selected from the group consisting of: a thickener, an emollient, an emulsifier, a humectant, a surfactant, a suspending agent, a film forming agent, a preservative, an antifoaming agent, a fragrance, a lower monoalcoholic polyol, a high boiling point solvent, a propellant, a colorant or a pigment.

Still further, in accordance with a preferred embodiment of the present invention, the carrier is additionally comprising an adjuvant within the carrier. In one preferred embodiment, said adjuvant is selected from the group consisting of an anti-oxidant, a metal sequestering agent, a buffering agent or mixtures thereof.

Moreover, in accordance with a preferred embodiment of the present invention, the carrier comprises at least one auxiliary agent, which triggers the release of the active agent from the microcapsule upon topical application. In certain embodiments, this auxiliary agent is selected from the group consisting of a surfactant, an electrolyte, a buffering agent, a high boiling point solvent, or mixtures thereof.

Additionally, in accordance with a preferred embodiment of the present invention, the composition further comprises an auxiliary vehicle, which is added to the composition prior to topical application to trigger the release of the active ingredient. In one preferred embodiment, the auxiliary vehicle comprises at least one member selected from the group consisting of a surfactant, an electrolyte, a buffering agent, a high boiling point solvent, or mixtures thereof.

Yet further, in accordance with a preferred embodiment of the present invention, the final form of the composition in the present invention, is selected from the group consisting of an emulsion, a cream, an aqueous solution, an oil, an ointment, a paste, a gel, a lotion, a milk, a suspension, a powder, an aerosol, a spray, a foam, a shampoo, a hair conditioner, a lacquer, a makeup, a solid stick, or a toothpaste.

Moreover, in accordance with a preferred embodiment of the present invention, the active ingredient present within the core of the microcapsules, or within the carrier, is selected from the group consisting of: a vitamin, an anti-inflammatory agent, an analgesic, an anti-fungal agent, an anti-biotic, an anti-viral agent, an anti-acne agent, an anti histamine, an enzyme, a co-enzyme, a humectant, a dermatological agent, an insect repellent, a perfume, a color, a dye, a skin whitening agent, an aromatic oil, a flavoring agent, a dental agent, or mixtures thereof.

In one preferred embodiment of the present invention, the active ingredient is present in an amount of about 0.001% to about 95% by weight of the microcapsule.

Additionally, in accordance with a preferred embodiment of the present invention, the microcapsular core further includes an adjuvant selected from the group consisting of an anti-oxidant, a metal-sequestering agent, a buffering agent, or mixtures thereof.

Further, in accordance with a preferred embodiment of the present invention, the core of the microcapsule is in a form selected from the group consisting of an emulsion, a solid, an oil solution, an aqueous solution, or a dispersion.

Still further, in accordance with a preferred embodiment of the present invention, said inorganic polymer comprising the microcapsular shell, is prepared from a sol-gel precursor selected from the group consisting of: a metal alkoxide monomer, a semi-metal alkoxide monomer, a metal ester monomer, a semi-metal ester monomer, a silazane monomer, a monomer of the formula M(R)n(P)ₘ, wherein M is a metallic or a semi metallic element, R is a hydrolyzable substituent, n is an integer from 2 to 6, P is a non polymerizable substituent and m is and integer from 0 to 6; or a partially hydrolyzed and partially condensed polymer thereof, or any mixture thereof.

Moreover, in accordance with a preferred embodiment of the present invention, the encapsulated active ingredient is a vitamin selected from the group consisting of vitamin C, an ester of vitamin C, a salt of vitamin C or mixtures thereof.

Additionally, in accordance with a preferred embodiment of the present invention, the composition is further comprising a metal sequestering agent within the microcapsular core, in addition to one of the following group: vitamin C, an ester of vitamin C, a salt of vitamin C or mixtures thereof. In certain embodiments, the metal sequestering agent is selected from the group consisting of ethylenediamine tetra acetic acid, hexamethylenediamine tetra acetic acid, ethylenediamine tetra(methylenephosphonic acid), diethylenetriamine penta (methylenephosphonic acid), or hexamethylenediamine tetra (methylene phosphonic acid), derivatives thereof, salts thereof, or mixtures thereof.

Further, in accordance with a preferred embodiment of the present invention, the encapsulated active ingredient is a retinoid. In accordance with a preferred embodiment of the present invention, the composition further comprises an anti oxidant within the microcapsular core, in addition to the retinoid. In one preferred embodiment, the antioxidant is selected from the group consisting of BHT, BHA, vitamin E, vitamin E acetate, vitamin E palmitate, vitamin C, an ester of vitamin C, a salt of vitamin C, or mixtures thereof.

Additionally, in accordance with a preferred embodiment of the present invention, the encapsulated dental agent is selected from the group consisting of sodium perborate, sodium percarbonate, or mixtures thereof.

Still further, in accordance with a preferred embodiment of the present invention, the encapsulated anti-acne agent or dental agent is a peroxide selected from the group consisting of benzoyl peroxide or urea peroxide.

Additionally, in accordance with a preferred embodiment of the present invention, the carrier further comprises a non-encapsulated ingredient, which is beneficial for treatment of the skin in the presence of benzoyl peroxide or urea peroxide. The non-encapsulated ingredient is capable of being oxidized in the presence of said peroxide. In one preferred embodiment, the non-encapsulated ingredient is selected from the group consisting of erythromycin, synthomycin, clindamycin, tetracycline, a retinoid, an alpha hydroxy acid, a salt thereof, a derivative thereof, or mixtures thereof.

Moreover, in accordance with a preferred embodiment of the present invention, the composition further comprises at least one ingredient selected from the group consisting of: a polymer, a fatty acid, a fatty acid derivative, a surfactant, a polysaccharide, a protein, a polypeptide, an amino acid, and a mixture thereof. This ingredient is present within the core or is attached to the shell of the microcapsules.

There is also provided in accordance with a preferred embodiment of the present invention, a process for the preparation of sol-gel microcapsules, comprising the steps of;
(a) preparing a hydrophobic solution or a hydrophobic dispersion comprising sol-gel precursors and an ingredient to be encapsulated;
(b) emulsifying the hydrophobic solution or dispersion of step (a) in an aqueous solution under high shear forces to obtain an emulsion;
(c) mixing and stirring the emulsion obtained, with a second aqueous solution, at a selected pH to obtain the sol-gel microcapsules.

In accordance with a preferred embodiment of the present invention, in the process of the present invention, the hydrophobic solution or dispersion further comprises a surfactant, a polymer, a polymeric surfactant, a suspending agent or mixtures thereof.

Additionally, in accordance with a preferred embodiment of the present invention, said sol-gel precursors are selected from the group consisting of: a metal alkoxide monomer, a semi-metal alkoxide monomer, a metal ester monomer, a semi-metal ester monomer, a silazane monomer, a monomer of the formula M(R)ₙ(P)ₘ, wherein M is a metallic or a semi metallic element, R is a hydrolyzable substituent, n is an integer from 2 to 6, P is a non polymerizable substituent and m is and integer from 0 to 6, a partially hydrolyzed and partially condensed polymer thereof, or any mixture thereof.

Moreover, in accordance with a preferred embodiment of the present invention, the hydrophobic dispersion is prepared by a method comprising the following steps:
(a) wetting and mixing a solid ingredient to be encapsulated with at list one additive selected from the group consisting of a liquid, a wetting agent, or a combination thereof;
(b) micronizing the solid by grinding or milling to obtain a dispersion of the solid within said additive.

Additionally, in accordance with a preferred embodiment of the present invention, the process further comprises adding at least one oil in step (a) or step (b) or in both steps (a) and (b) to obtain a dispersion of the solid within the oil.

Moreover, in accordance with a preferred embodiment of the present invention, the process further comprises the step of adding and mixing a sol-gel precursor in step (a) or (b), or adding and mixing the sol-gel precursor in both steps (a) and (b).

Still further, in accordance with a preferred embodiment of the present invention, the process further comprises the step of adding and mixing a sol-gel precursor with the obtained dispersion.

Additionally, in accordance with a preferred embodiment of the present invention, the process further comprises the step of adding and mixing with at least one oil to obtain a dispersion of the solid within the oil.

Further, in accordance with a preferred embodiment of the present invention, in the process of the present invention, the hydrophobic dispersion is prepared by a method comprising the following steps:
(a) micronizing a solid ingredient to be encapsulated by grinding or milling;
(b) wetting and mixing a solid ingredient to be encapsulated with at list one additive selected from the group consisting of a liquid, a wetting agent, or a combination thereof. In one preferred embodiment, this method further comprises the step of adding at least one dispersing phase selected from the group consisting of an oil, a sol-gel precursor or a combination thereof and mixing to obtain a dispersion.

Still further, in accordance with a preferred embodiment of the present invention, the concentration of the solid is approximately 0.001 % to 95% (wt./wt.) based on the total weight of the solid and oil.

Moreover, in accordance with a preferred embodiment of the present invention, the concentration of the solid in the final dispersion is between about 1 to about 95% (wt./wt.)

Additionally, in accordance with a preferred embodiment of the present invention, the particle size of the dispersed solid is between about 0.1 to about 20 micron.

Moreover, in accordance with a preferred embodiment of the present invention, the concentration of the oil in the final dispersion is between about 5 to about 99% (wt./wt.).

Additionally, in accordance with a preferred embodiment of the present invention, the liquid is selected from the group consisting of a hydrophobic liquid, a hydrophilic liquid, an aqueous liquid, or mixtures thereof.

Further, in accordance with a preferred embodiment of the present invention, the hydrophobic liquid is selected from the group consisting of an oil, a sol-gel precursor, or mixtures thereof.

Still further, in accordance with a preferred embodiment of the present invention, the concentration of the sol-gel precursors in the final dispersion is between about 5 to about 99% (wt./wt.).

Moreover, in accordance with a preferred embodiment of the present invention, the hydrophilic liquid used as a wetting agent is glycerol, and the aqueous liquid used as a wetting agent is water. In another preferred embodiment, the wetting agent is selected from the group consisting of: a surfactant, a polymeric surfactant, or mixtures thereof. In accordance with a preferred embodiment of the present invention, when the wetting agent is a surfactant, the concentration of surfactant in the final dispersion is preferably between about 0.1 to about 20% (wt./wt.). In accordance with a preferred embodiment of the present invention, the surfactant is selected from the group consisting of an anionic surfactant, a cationic surfactant, an amphoteric surfactant, a nonionic surfactant, or mixtures thereof.

Further, in accordance with a preferred embodiment of the present invention, the polymeric surfactant used as a wetting agent is selected from the group consisting of: an anionic polymeric surfactant, a cationic polymeric surfactant, an amphoteric polymeric surfactant, a nonionic polymeric surfactant, a hydrocarbon-based polymer, a silicone polymer or mixtures thereof.

Moreover, in accordance with a preferred embodiment of the present invention, the hydrocarbon-based polymer includes at least one ionic or non-ionic functional group selected from the group consisting of: a phosphate ester, a sulfate, a carboxylate, a sulfosuccinate, a sulfonate, a thiosulfonate, an amino propionate, a betaine, a phosphobetaine, an alkyl quaternary compound, an amido quaternary compound, an imidazoline quaternary compound, a carboxy quaternary compound, an alcohol aloxylate, an alkanolamide, an ester.

Additionally, in accordance with a preferred embodiment of the present invention, the silicone polymer used as a wetting agent, is selected from the group consisting of a silicone phosphate ester polymer, a silicone sulfate polymer, a silicone carboxylate polymer, a silicone sulfosuccinate polymer, a silicone sulfonate polymer, a silicone thiosulfate polymer, a silicone amphoteric polymer, a silicone betaine polymer, a silicone phosphobetaine polymer, a silicone alkyl quaternary polymer, a silicone quaternary polymer, a silicone imidazoline quaternary polymer, a silicone carboxy quaternary polymer, a dimethcone copolyol polymer, a silicone alkanolamide polymer, a silicone ester polymer, and a mixture thereof.

Additionally, in accordance with a preferred embodiment of the present invention, in the process of the present invention, the oil is selected from a group consisting of mineral oil, dimethicone, cyclomethicone, alkyl siloxanes, alkylether siloxanes, dimethicone copolyols, C12-15 Alkyl Benzoate, Isostearyl Benzoate, PPG-15 Stearyl Ether Benzoate, Octyldodecyl Benzoate, Stearyl Benzoate, Methyl Gluceth-20 Benzoate, Poloxamer 182 Dibenzoate, Poloxamer 105 Benzoate, Transcutol, Bernel Ester, Diethylhexylmaleate, Diethylhexylsebacate, Diethylhexyladipate, Diisopropyladipate, Diisopropylsebacate, Diisopropylmaleate, Ethylhexylsalicylate, Tridecylsalicylate, Butiloctylsalicylate, Isopropylmyristate, or mixtures thereof.

### BRIEF DESCRIPTION OF THE DRAWINGS

The present invention is herein described, by way of example only, with reference to the accompanying drawings, wherein:
Figure 1 is a graph illustrating the amount of octylmethoxy cinnamate (OMC) released from a suspension of microcapsules which contain OMC, over a period of 22 hours.
Figure 2 is a graph illustrating the amount of OMC released during the first six hours, from a suspension of microcapsules which encapsulate OMC.

### DETAILED DESCRIPTION OF THE INVENTION

It is appreciated that the detailed description that follows is intended only to illustrate certain preferred embodiments of the present invention. It is in no way intended to limit the scope of the invention, as set out in the claims.

The present invention discloses a composition for use in cosmetic or pharmaceutical formulations, in which one or more active ingredients are encapsulated within microcapsules that are designed to release their contents after topical application. Encapsulation of a sensitive active ingredient in sol-gel capsules can protect it from other ingredients in the formulation and from the environment, and thus extends the shelf life of the end-product. After topical application, however, the microcapsules break and thus, release and deliver the active ingredient to the skin.

In a preferred embodiment of the present invention, the microcapsules are designed to break upon application to the skin or hair, due to their relatively large size, 3-50 micron diameter, which imparts them with structural weakness. Friction is formed upon application to the skin, which is sufficient to shatter their relatively fragile shell wall. (The thickness of the shell wall is approximately 100 nm, which is not relatively thin; the fragility of the microcapsules is due, rather, to the large diameter of the capsules.)

In the present invention the applicants have succeeded in controlling the microcapsule size in a pre-determined manner, a feat which has not been accomplished in prior art, and is of technical importance. The timing of the topical release of the active ingredient can now be programmed, and a diversified release profile can be attained. For instance, microcapsules of varied sizes can be included in one composition, so that the active ingredient is released over a period of time. In another example, microcapsules containing one active ingredient are released at one time-point after application, then microcapsules of another size release a second active ingredient at a second time point, giving a rich therapeutic or cosmetic effect. The release of active ingredient can be designed to be immediate, or sustained; this can be controlled by varying the composition of the microcapsular shell, its diameter, and by varying the composition of the carrier surrounding the microcapsules.

In one embodiment, the microcapsules of the present invention are designed to break after application due to the nature of a carrier which surrounds them. In one preferred embodiment, this carrier has a water content of 80-99%. After topical application, the water evaporates, leaving the capsules exposed to the environment. The microcapsules dry and break, releasing their contents. In another embodiment, the carrier contains a solvent with a high boiling point, in addition to the high water content. The solvent is approximately 10% by wt. of the carrier content. In this design, the active ingredient is not released due to breakage of the microcapsules, rather, after application the water evaporates, and the solvent can penetrate the microcapsular shell, to dissolve and extract the active ingredient from within the microcapsules.

In another embodiment, the encapsulated material may be extracted by moisture or electrolytes present on skin, sweat and sebum. Alternatively, the end-product can undergo a change in its composition upon application, for example, by addition of another composition which may contain electrolytes, surfactants, or a buffering agent, that trigger the release.

Additives which are capable of maintaining humidity and moisture can be added during preparation of the capsules to control the drying process. Such additives may be entrapped in the sol-gel pores, or may be covalently attached to the sol-gel precursors through a non-hydrolyzable residue. Organic polymers and/or surfactants may be added while the sol-gel matrix is being formed, to control the surface nature of the matrix and the rate of diffusion through the pores. Since the microcapsular shell may be composed of primary sol sub-micron particles, the effective pore size of the shell may be controlled by electrolytes, due to electrostatic interactions. This may be a trigger for release of the active ingredients.

The active ingredient may be encapsulated alone, or with other ingredients within the same capsule. Co-encapsulation of compounds that enhance stability of the sensitive ingredient is beneficial. For example, anti oxidants can be co-encapsulated with oxygen-sensitive or oxidant-sensitive ingredients, to give "localized protection". Similarly, base-sensitive actives may be co-encapsulated with proton donating compounds that can act as a local buffer source. Acid-sensitive active ingredients can be co-encapsulated with proton acceptors, in order to protect them. Water-sensitive actives may show improved stability by encapsulating them as a solute in a hydrophobic, water repelling oil. Co-encapsulating with sunscreen active ingredients, can protect light sensitive compounds. Co-encapsulation of a sensitive ingredient and a protective ingredient in one capsule, augments the efficacy of the protecting ingredient as both ingredients are encased together in the capsule. Moreover, by constructing such an organized system, the overall concentration of protecting ingredient, which is present in the composition, can be reduced significantly.

Since the encapsulation creates micro-domains within the entire formulation, one active ingredient can be encapsulated while a second active ingredient can be present in the carrier that surrounds the microcapsules. This is advantageous when the ingredients acts synergistically together, yet one is chemically reactive with another. For instance, benzoyl peroxide, retinoids and certain anti-biotics are all beneficial for the treatment of acne, yet cannot be formulated together since the peroxide would oxidize the other active ingredients. Therefore, benzoyl peroxide may be encapsulated within sol-gel microcapsules, and another active ingredient can be present in the pharmaceutical carrier.

The capsules can be easily incorporated in cosmetic or pharmaceutical compositions. Capsules that have a hydrophilic external surface can be dispersed in water phases, while capsules which have a hydrophobic external surface can be dispersed in oil phases. In both instances, simple mixing is sufficient to achieve effective dispersion. The final product may take the form of an oil, a gel, a solid stick, a lotion, a cream, a milk, an aerosol, a spray, a powder, a foam, a shampoo, a hair conditioner, a lacquer or a make-up. The microcapsules are not heat sensitive, and unless the encapsulated active is heat sensitive they may be heated to temperatures normally used in formulation of cosmetic compositions, as is accepted in the art (for instance, up to a maximum temperature of 80°C, for up to 2 hours).

The sol-gel process is adaptable to different encapsulating materials, including pure silica, organically modified silica, titania, silizane, zirconia, alumina, and others, as well as combinations of the above. Thus, the character of the shell can be modified to suit the needs of a specific application.

Preferably, the loading of active ingredient in the capsules is in the range of approximately 0.001% to 95% by wt. of the microcapsule, more preferably in the range of approximately 5-80% by wt. of the microcapsule. The character of the core depends on the process of microcapsule preparation, the percentage of active ingredient present in the core, and the solubility of the active ingredient in the core. For example, a higher percentage of a solid active ingredient in the core will result in a core which is more viscous.

Microcapsules of 0.1 to 100µ diameter are obtained. More preferably particles of 3 to 50µ, or most preferably 8-50µ in diameter, are obtained. The microcapsules obtained are cosmetically and pharmaceutically acceptable, being smooth and optionally transparent, but are large enough so that they can not penetrate the epidermis.

In the present invention, the sol-gel microcapsules are prepared using the following steps:
(a) preparing a hydrophobic solution or a hydrophobic dispersion comprising sol-gel precursors and an ingredient to be encapsulated;
(b) emulsifying the hydrophobic solution or dispersion of step (a) in an aqueous solution under high shear forces to obtain an emulsion.
(c) mixing and stirring the emulsion obtained, with a second aqueous solution, at a selected pH to obtain the sol-gel microcapsules.

Oil-soluble active ingredients to be encapsulated, are first dissolved within at least one oil or within the sol-gel precursor, to form a hydrophobic solution. An active ingredient that does not dissolve in a hydrophobic liquid may alternatively be encapsulated as a dispersion, by first dispersing it in a suitable oil, which is miscible with the sol-gel precursor.

The hydrophobic solution or dispersion may further comprise a surfactant, a polymer, a polymeric surfactant, a suspending agent or mixtures thereof.

According to one method, the hydrophobic dispersion may be prepared by first wetting and mixing a solid ingredient to be encapsulated with at list one additive which may be a liquid, a wetting agent, or a combination thereof.

Next, in the second step of the process, the solid is micronized by grinding or milling to obtain a dispersion of the solid within the additive. A roller mill, a ball mill, a colloid mill a high-pressure mill, or a high-shear mill may be employed for this purpose. The milling or grinding is continued, until a dispersion of the active ingredient at the desired particle size, is obtained, within the liquid phase.

The liquid used for wetting may be a hydrophobic liquid, a hydrophilic liquid, an aqueous liquid or a combination thereof. The hydrophobic liquid may be, for example, an oil or a sol-gel precursor. The same oil used in the wetting step, may also be used later in an additional, optional step, for dispersing the solid.

When high concentrations of an ingredient are used, to obtain high loading of the active ingredient in the microcapsule, the amount of oil used in the wetting stage may be sufficient, and no additional quantity of oil may be required at a later stage, to form the final dispersion. The dispersion formed in the wetting step will be a highly concentrated slurry.

The wetting agent used in the wetting step may be, for example, a surfactant, a polymeric surfactant or mixtures thereof. Alternatively, glycerol, water or organic solvents may also be used in the wetting step.

Optionally, in a variation on this method, an oil used as the dispersing phase may also be added during the first or second step, before or after the micronization. Thus a dispersion of the solid within the oil is obtained.

A sol-gel precursor may be added during the first or second step, before or after the micronization.

According to another method, the hydrophobic dispersion may be prepared using the following steps (which represent a reversal of the main steps of the above-mentioned method):
In the first step the solid ingredient to be encapsulated is micronized by grinding or milling.
In the second step the solid ingredient to be encapsulated is wetted and mixed with at list one additive which may be a liquid, a wetting agent, or a combination thereof.

The process may further comprise the step of adding at least one dispersing phase selected from the group consisting of an oil, a sol-gel precursor or a combination thereof; and mixing to obtain a dispersion.

When high concentrations of an ingredient are used to obtain high loading of the active ingredient in the microcapsule, the amount of oil used in the wetting stage may be sufficient and no additional quantity of oil may be required at any later stage, to form the final dispersion. In this case, the dispersion formed in the wetting step is a highly concentrated slurry.

Preferably, the concentration of solid active ingredient, to be encapsulated as a dispersion in the core of the end-product microcapsule, is approximately 1 to 95% wt./wt, as measured immediately before emulsification under high shear force. Most preferably, this concentration is between 20 to 50% wt./wt. In addition, the particle size of the dispersed solid is preferably approximately 0.1 to 20 micron, more preferably between 0.2 to 2 micron. Preferably, the concentration of surfactant at this stage (immediately before emulsification) is between about 0.1 to about 20% wt., most preferably between 1 to 10% wt. Moreover, the preferred concentration of oil in at this point, is between about 5 to about 99% wt., most preferably between 10 to 40% wt. Preferably, the concentration of the sol-gel precursors in the dispersion immediately prior to emulsification, is about 5 to about 99% wt., preferably between 20 to 60% wt. Preferably, the concentration of the solid active ingredient out of the total (solid + oil) weight, is approximately 0.001-95% wt., more preferably 40-90% wt.

The sol-gel precursors can be selected from metal or semi-metal alkoxide monomers, metal ester monomers, silazane monomers, semi-metal ester monomers or monomers of the formula M(R)ₙ(P)ₘ, wherein "M" is a metallic or semi metallic element, "R" is a hydrolyzable substituent, "n" is an integer from 2 to 6, "P" is a non polymerizable substituent and "m" is an integer from 0 to 6. Alternatively, a partially hydrolyzed and partially condensed polymer thereof may be used, or any mixture thereof.

In a preferred embodiment of the present invention, the sol-gel microcapsules are silica or organically modified silica microcapsules. The term "organically modified silica microcapsules" refers to sol-gel microcapsules which are obtained when the sol-gel precursors are of the formula M(R)ₙ(P)ₘ, wherein "M" is Si, "R" is a hydrolyzable substituent, "n" is an integer from 2 to 5, "P" is a non polymerizable substituent and "m" is an integer from 1 to 6.

Based on the nature of the active ingredient undergoing encapsulation; the reaction is performed under conditions chosen so as to protect it from decomposition, i.e. inert atmosphere, protection from light, reaction in the presence of oxygen scavengers or metal ion sequestering agents, etc.

The final form of the product can be a dry powder containing free-flowing microcapsules. A dry powder is obtained, despite the fact that the microcapsules themselves enclose up to 95% by wt. of oils.

The active ingredients which can benefit from encapsulation, are those that are sensitive to oxygen or to components present in the pharmaceutical or cosmetic carrier. In addition, ingredients which are irritating to the skin may be encapsulated, thus limiting the time period to which the skin is exposed to them. These can include members of the following types of ingredients: vitamins, anti-inflammatory agents, analgesics, anti-fungal agents, anti-biotics, anti-viral agents, anti-parasitic agents, anti-acne agents, humectants, dermatological agents, enzymes and co-enzymes, insect repellents, perfumes, aromatic oils, colors, dyes, skin whitening agents, flavoring agents, or dental agents.

Non-limiting examples of members of each of these groups include vitamins, such as vitamin A and its analogs and derivatives: retinol, retinal, retinyl palmitate, retinoic acid, tretinoin, iso-tretinoin (known collectively as retinoids), vitamin E (tocopherol and its derivatives), vitamin C (L-ascorbic acid and its esters and other derivatives), vitamin B₃ (niacinamide and its derivatives), alpha hydroxy acids (for example glycolic acid, lactic acid, tartaric acid, malic acid, citric acid, etc.), beta hydroxy acids (for example salicylic acid and the like).

Skin bleaching agents include hydroquinone, monobenzone and others; dental agents include the bleaching agents urea peroxide, benzoyl peroxide, sodium perborate and sodium percarbonate; other dermatological active ingredients useful in cosmetic applications include jojoba oil and aromatic oils (such as methyl salicylate, wintergreen, peppermint oil, bay oil, eucalyptus oil, citrus oils, etc.). Additional ingredients for cosmetic application are enzymes and co-enzymes, such as co-enzyme Q10, papain enzyme, lipases, proteases, superoxide dismutase, fibrinolysin, desoxyribonuclease, trypsin, collagenase, sutilains and others.

Yet other types of active ingredients that are beneficial to encapsulate in the microcapsules of the present invention, include humectants (such as glycerol, sodium pyroglutamate, ornithine), and additional dermatological agents (ammonium phenolsulfonate, bismuth subgallate, zinc phenolsulfonate and zinc salicylate).

Analgesics and anti-inflammatories can likewise benefit from encapsulation. Anti-inflammatories include for example, methyl salicylate, aspirin, ibuprofen, and naproxen. Additional anti-inflammatories useful in topical applications, are corticosteroids, including for instance, flurandrenolide, clobetasol propionate, halobetasol propionate, fluticasone propionate, betamethasone dipropionate, betamethasone benzoate, betamethasone valerate, desoximethasone, dexamethasone, diflorasone diacetate, mometasone furoate, amcinodine, halcinonide, fluocinonide, fluocinolone acetonide, desonide, triamcinolone acetonide, hydrocortisone, hydrocortisone acetate, fluoromethalone, methylprednisolone, and predinicarbate. Anti-infectious and anti acne agents include benzoyl peroxide, sulfur, resorcinol, salicylic acid. Antifungal agents include miconazole, clotrimazole, butoconazole, fenticonasole, tioconazole, terconazole, sulconazole, fluconazole, haloprogin, ketonazole, ketoconazole, oxinazole, econazole, itraconazole, terbinafine, nystatin, griseofulvin. Antiviral agents include famciclovir, valacyclovir, acyclovir; antibiotics include erythromycin, clyndamycin, synthomycin, tetracycline, metronidazole and the likes. Anti-parasitic agents can include scabicedes, for example permethrin, crotamiton, lindane, ivermectin, and insect repellents such as pediculicides for treatment of lice, for example pyrethrins, permethrin, malathion, lindane and the likes. Antihistamines include, for instance, chlorpheniramine, brompheniramine, dexchlorpheniramine, tripolidine, clemastine, diphenhydramine, promethazine, piperazines, piperidines, astemizole, loratadine, and terfenadine.

Local anasthetics include for example, benzocaine, butamben, butamben picrate, cocaine, procaine, tetracaine, lidocaine, pramoxine hydrochloride. Chemotherapeutic agents include 5-fluorouracil, masoprocol, mechlorethamine, cyclophosphamide, vincristine, chlorambucil, streptozocin, methotrexate, bleomycin, dactinomycin, daunorubicin, coxorubicin, tamoxifen.

Another type of ingredient that is frequently added to the composition and which may cause complications in formulation is fragrance. While having no therapeutic action, fragrances often cause skin irritation. Entrapment of fragrances may serve to decrease skin sensitivity to fragrances, while extending their effectiveness period through sustained release.

Other types of active ingredients are insect repellants, colors, and dyes. Colors and dyes are incompatible with formulation ingredients and can be protected by encapsulation, and released upon application. Examples of flavoring agents are methyl salicylate, and peppermint oil, which can be formulated, for example within a composition useful for dental application.

While the invention has been described with respect to a limited number of embodiments, it will be appreciated that many variations, modifications and other applications of the invention may be made.

Reference is now made to the following examples, which are intended to illustrate the invention, in a non-limiting manner.:

### Example 1: Encapsulation of methyl salicylate in silica

Methyl salicylate is useful as a topical anti inflammatory agent, and as a flavoring agent in dental care products.

33g of methyl salicylate was mixed with 33g tetraethoxy silane (TEOS). The organic phase was emulsified in 300g of aqueous solution containing 1% cetyltrimethyl ammonium chloride (CTAC) under high shear. The vessel walls were cooled by immersion in an ice-water bath during the homogenizing process. This emulsion was then poured into an IKA LR-A 1000 Laboratory reactor, equipped with Eurostar Power control-visc. P4 stirrer, containing 300g NaOH aqueous solution at pH 11.5. The solution was stirred at 200 rpm. After 7 days the product was precipitated in a centrifuge. The final product was re-suspended in water containing 1% polyvinyl pyrrolidon to receive a suspension containing 32.4% methyl salicylate encapsulated in silica particles of 0.5 to 10 micron.

### Example 2: Encapsulation of methyl salicylate and oleic acid in silica

Methyl salicylate degrades through hydrolysis in a basic environment. To protect it from hydrolysis it is co-encapsulated with oleic acid.

8.25g methyl salicylate are mixed with 24.25g oleic acid. 33g TEOS are added to the mixture. This oil phase is emulsified and the emulsion is poured into a basic solution of pH 11.5. The mixture is stirred at 50 to 240 rpm. After 3 days, 6.4 g of MgSO₄ are added to 440 g of the resulting suspension and mixed for 1 hour, and allowed to settle for 2 hours. It is then filtered using a Whatman No.40 filter.

The product obtained is a paste containing 5.6 % methyl salicylate.

### Example 3: Encapsulation of Erythromycin in silica

Combinations of erythromycin and benzoyl peroxide are useful in the treatment of acne but usually must be formulated as a two component system, because of incompatibility of the two active ingredients.

1.7g erythromycin is mixed with 14.9g octylmethoxy cinnamate. 19.5g TEOS are added to the mixture. This oil phase is emulsified and the emulsion is poured into a basic solution of pH 11.5. The mixture is stirred at 50 to 240 rpm. Flocculation is induced by the addition of MgSO₄ at a final concentration of 0.1% by weight. The precipitate is collected by filtration with a Whatman No. 40 paper. The product obtained is a paste, with a particle size distribution of 1-12 micron (an average size of 6.2 micron).

### Example 4: Encapsulation of Benzoyl Peroxide in Silica

Benzoyl Peroxide is useful as a topical anti-acne agent.

30g of 7 % (w/w) benzoyl peroxide (BZP) in diisopropylsebacate ester was mixed with 20g of TEOS. The organic phase was emulsified in 200g. of an aqueous solution containing 1% CTAC under high shear. The vessel walls were cooled by immersion in an ice-water bath during the homogenizing process. This emulsion was then poured into an IKA LR-A 1000 Laboratory reactor, equipped with Eurostar Power control-visc P4 stirrer, containing 200g NaOH aqueous solution at pH 10. The solution was stirred at 200 rpm. After 3 days the product was separated and washed. The final product was re-suspended in water to obtain a dispersion containing a 3% benzoyl peroxide encapsulated in silica particles of 0.5-15 micron.

### Example 5: Encapsulation of Benzoyl Peroxide in Silica

32.5g of micronized benzoyl peroxide containing 25% water (w/w) was dispersed by a Polytron homogenizer in a solution containing 1g silicon emulsifier (Abil EM90, Goldschmit), 5g volatile silicon oil (Dow Coming 200(R) Fluid, 0.65cst) and 11.5g TEOS. The dispersion obtained was poured into 200 gr. of an aqueous solution containing 1% CTAC under mild stirring conditions using a conventional propeller agitator. The s/o/w emulsion obtained was then poured into an IKA LR-A 1000 Laboratory reactor, equipped with Eurostar Power control-visc P4 stirrer, containing 200g NaOH aqueous solution at pH 10. The solution was stirred at 200 rpm. After 3 days the product was separated and washed. The final product was re-suspended in water to obtain a dispersion containing a 35% benzoyl peroxide encapsulated in silica particles of 0.5 - 50 micron.

### Example 6: Breaking of capsules by force

A drop of a silica dispersion as in Example 4 was put between two slides of glass. Spheres of about 10 micron were observed under the microscope (X 100 magnification). The top glass was pressed several times to the bottom glass using a hand-held plastic rod, and the slide was re-examined microscopically. Broken spheres were observed. The spherical structures were distorted.

### Example 7: Release of encapsulated matter through drying of the capsules

An encapsulated sunscreen compound, octylmethoxy cinnamate (OMC) is used as a model in this example, and its level of passage is followed, from within a suspension containing it, through a membrane into a glass cell which together, simulate physiological conditions on the epidermis.

Two hydrophobic Tuffryn® membranes are pretreated with isopropyl myristate, and each membrane is placed over the opening of a glass cell (a horizontal Franz-type cell). The area of each membrane is 1.3cm². The upper side of the membrane is exposed to the air, and OMC suspension can be applied to it. The underside of the membrane faces the contents of the glass cell, which hold approximately 3.5ml of a 4% (w/v) bovine serum albumin (BSA) in PBS, at pH 7.4, at a temp. of 35°C. The PBS-BSA solution is continuously stirred with Teflon-coated magnets.

A suspension of microcapsules containing 8.8% OMC (octylmethoxy cinnamate) is gently applied over each membrane and spread evenly on its surface. One membrane receives 0.4ml/cm² of OMC suspension, while the second membrane receives 0.005ml/cm² of OMC suspension.

At several time points over a period of 22 hours, a sample of 200µl is removed from the solution below the membrane, and analyzed using HPLC to determine the amount of OMC which was released from the microcapsules and passed through the membrane into the glass cell. After each sample is removed, an equal volume of fresh solution is returned to the cell.

Cumulative receptor concentration-time profiles are then plotted and used to compare the effect of concentration on passage through the membrane.

Refer now to Table 1, which details the amount of OMC present in a sample taken from below the membrane after passage through each membrane, as measured by HPLC in units of µg/cell.

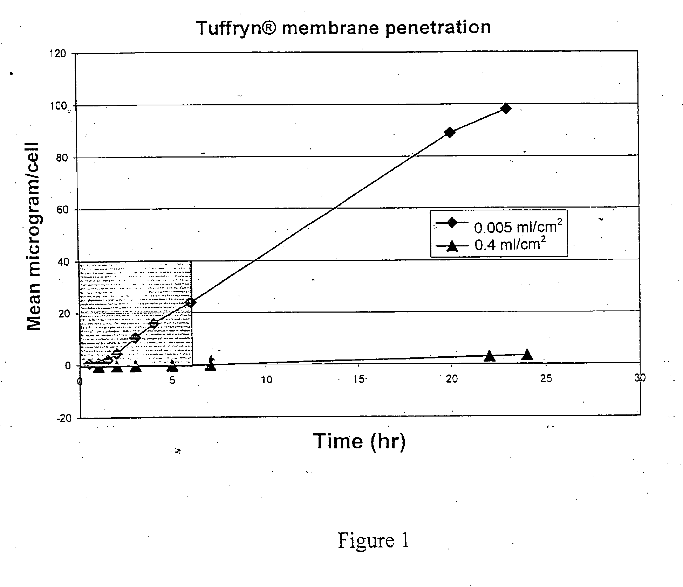

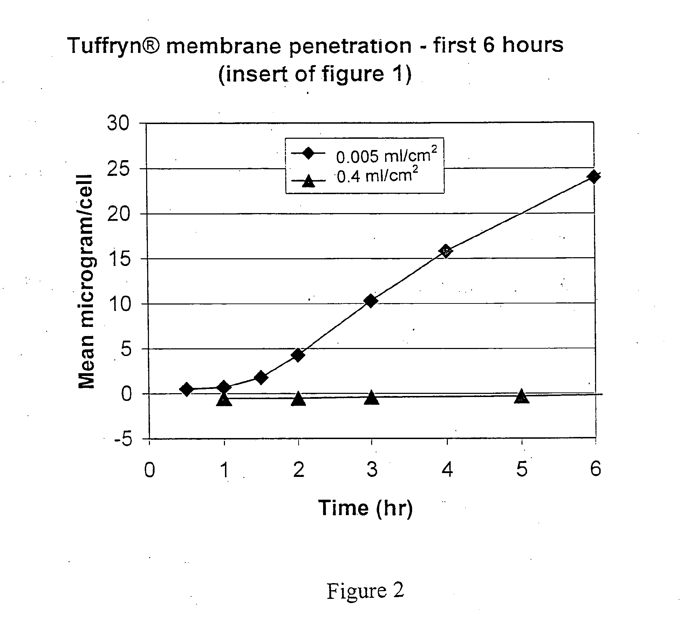

Refer as well to Figure 1, which plots these results in graph format, and to Figure 2, which shows the first six hours of the experiment on a graph having an enlarged scale. (Figure 2 represents an enlargement of the grayed area in the lower left-hand corner of Figure 1.)

After 1.5 hour, the OMC was released at significantly increasing amounts through the membrane which received 0.005ml/cm² of OMC. Throughout all time-points of the experiment, the higher dose of 0.4ml/cm² did not release any significant amount through the membrane. This is contrary to the expectation, as the higher dose of 0.4ml/cm² contains about 80 times more OMC as compared to the lower dose of 0.005ml/cm². These paradoxical results can be understood, if the release of OMC from within the microcapsules is triggered by total evaporation of the water content, from the silica suspension, in the case of 0.005ml/cm². At that dosage, the amount of water is minimal, and the suspension is highly exposed to the air above the membrane, and thus, subject to evaporation. The total evaporation of water leads to drying of the silica pores, which results in release of the encapsulated matter. This drying does not occur in the higher dose of 0.4ml/cm², therefore the capsules retain their contents, and no OMC is passed through the membrane. Tailoring of the formulation of the carrier surrounding the microcapsules, to induce certain drying rates can therefore be used as a mechanism of controlling the release of the encapsulated matter.

**Table 1: Amount of OMC Released Over Time, Through A Membrane Applied With 0.4ml/cm² or 0.005ml/cm²of OMC.**

| **0.005ml/cm²** | | | | |
|---|---|---|---|---|
| Time | Sample 1 | Sample 2 | Sample 3 | Mean data |
| (hours) | (µg/cell) | (µg/cell) | (µg/cell) | ±SEM (µg/cell) |
| 0.5 | 0.5 | 0.4 | 0.6 | 0.5±0.0 |
| 1 | 1.0 | 0.5 | 0.6 | 0.7±0.1 |
| 1.5 | 3.6 | 0.9 | 0.7 | 1.8±0.9 |
| 2 | 7.5 | 3.3 | 2.1 | 4.3±1.6 |
| 3 | 15.9 | 8.4 | 6.6 | 10.3±2.8 |
| 4 | 20.8 | 14.1 | 12.6 | 15.8±2.5 |
| 6 | 28.7 | 22.2 | 21.2 | 24.0±2.3 |
| 20 | 61.3 | 96.7 | 109.1 | 89.0±14.3 |
| 23 | 66.8 | 106.0 | 121.7 | 98.1+16.3 |
| | | | | |

| **0.4ml/cm²** | | | | |
|---|---|---|---|---|
| Time | Sample 1 | Sample 2 | Sample | Mean data |
| (hours) | (µg/cell) | (µg/cell) | 3 (µg/cell) | ±SEM (µg/cell) |
| 1 | -0.5 | -0.5 | -0.5 | -0.5±0.0 |
| 2 | -0.5 | -0.5 | -0.5 | -0.5±0.0 |
| 3 | -0.5 | -0.5 | -0.4 | -0.4±0.0 |
| 5 | -0.3 | -0.4 | -0.2 | -0.3±0.1 |
| 7 | -0.1 | -0.3 | -0.1 | -0.1±0.1 |
| 22 | 5.3 | 1.5 | 1.9 | 2.9±1.2 |
| 24 | 6.1 | 1.6 | 2.1 | 3.3±1.4 |

### Example 8: Stabilization of oxidation-sensitive compound by encapsulation of benzoyl peroxide

It is desired to formulate together benzoyl peroxide and oxidation-sensitive active ingredients like retinoids and antibiotics for the treatment of acne. Encapsulation of benzoyl peroxide can facilitate obtaining a stable formulation containing both ingredients.

An azo dye, Congo Red (CR) is used a model of oxidation-sensitive compound in this test. Three comperative systmes are made in this test. In the first system, 9g of silica dispersion of Example 4 containing 4% (w/w) encapsulated benzoyl peroxide and 9g water were stirred in a 25 ml beaker with a mgnetic stirrer. In the second system, 9g aquesous dispersion containing 4% (w/w) free benzoyl peroxide suspended with 1% (w/w) Tween 20 and 9g water were stirred in a 25 ml beaker with a mgnetic stirrer. The third system is a blank for the experiment. 9g 1% (w/w) Tween 20 and 9g water were stirred in a 25 ml beaker with a mgnetic stirrer.

2ml 0.02% (w/w) CR solution were added to each system at time 0. After 1 minute stirring a 1.5 to 2ml sample was taken from the stirred suspension, filtered through a 0.2 micron cut-off hydrophilic filter into a spectrophotometer quartz quevette and the spectrum was taken over the range of 200 to 700nm. Additional samples were taken at 30, 50 and 75 minute using the same method, and the spectrum was measured.

It was found that the λₘₐₓ of CR was shifted as compared to its spectrum in water: the maxumum in water is 497nm, it is 509nm in the free benzoyl peroxide and blank system due to the presence of Tween 20, and it is 470nm in the encapsulated benzoyl peroxide system due to the presence of CTAC. Thus, each system was followed at its respective λₘₐₓ.

It was found that in the system containing free benzoyl peroxide CR is oxidized, as seen from the gradual depletion of the absorbance, to the extent that after 75 minutes 80% of the original amount was depleted. No change in absorbance was observed in the system containing encapsulated benzoyl peroxide or in the blank system.

It is concluded from these results that benzoyl peroxide can be effectively encapsulted in sol-gel microcapsules so that it can be co-formulated with oxidation-sensitive compounds. The sensitive conpounds are not oxidized by the encapsulted benzoyl peroxed while in formulation.

From Examples 1-7 it is apparent that unstable active ingredients can be encapsulated in microcapsules, and a composition for topical application can be formed, according to the present invention. The active ingredient can be encapsulated alone, or can be co-encapsulated with a stabilizing material, as in Example 2. The composition can be designed so that the microcapsules will release their contents after topical application, by using, for example, a high water content within the carrier surrounding the microcapsules (as in Example 7), or any other release mechanism.

## Claims

1. A process for the preparation of microcapsules having a core-shell structure, wherein said microcapsules have a diameter of approximately 0.1 to 100 micron, wherein each core includes at least one active ingredient, said core is encapsulated within a microcapsular shell, and wherein said shell is comprised of at least one inorganic polymer obtained by a sol-gel process, and said microcapsular shell protects the active ingredient prior to topical application and releases said active ingredient after topical application, said process comprises the steps of; (a) preparing a hydrophobic solution or a hydrophobic dispersion comprising sol-gel precursors and an ingredient to be encapsulated; (b) emulsifying the hydrophobic solution or dispersion of step (a) in an aqueous solution under high shear forces to obtain an emulsion; (c) mixing and stirring the emulsion obtained, with a second aqueous solution, at a selected pH to obtain the sol-gel microcapsules.

2. The process of claim 1, wherein said hydrophobic solution or dispersion further comprises a surfactant, a polymer, a polymeric surfactant, a suspending agent or mixtures thereof.

3. The process of claim 1, wherein said sol-gel precursors are selected from the group consisting of a metal alkoxide monomer, a semi-metal alkoxide monomer, a metal ester monomer, a semi-metal ester monomer, a silazane monomer, a monomer of the formula M(R)ₙ(P)ₘ, wherein M is a metallic or a semi metallic element, R is a hydrolyzable substituent, n is an integer from 2 to 6, P is a non polymerizable substituent and m is and integer from 0 to 6, a partially hydrolyzed and partially condensed polymer thereof, or any mixture thereof.

4. The process of claim 1, wherein said hydrophobic dispersion is prepared by a method comprising the following steps: (a) wetting and mixing a solid ingredient to be encapsulated with at list one additive selected from the group consisting of a liquid, a wetting agent, or a combination thereof; (b) micronizing the solid by grinding or milling to obtain a dispersion of the solid within said additive.

5. The process of claim 4, further comprising adding at least one oil in step (a) or step (b) or in both steps (a) and (b) to obtain a dispersion of the solid within the oil.

6. The process of claim 1, wherein said hydrophobic dispersion is prepared by a method comprising the following steps: (a) micronizing a solid ingredient to be encapsulated by grinding or milling; (b) wetting and mixing a solid ingredient to be encapsulated with at list one additive selected from the group consisting of a liquid, a wetting agent, or a combination thereof.

7. The process of claim 6, further comprising the step of adding at least one dispersing phase selected from the group consisting of an oil, a sol-gel precursor or a combination thereof and mixing to obtain a dispersion.

8. The process according to claim 0-4, wherein the concentration of the solid is approximately 0.001 percent to 95 percent (wt./wt.) based on the total weight of the solid and oil.

9. The process according to claim 4, wherein the particle size of the dispersed solid is between about 0.1 to about 20 micron.

10. The process according to claim 4, wherein an oil is present in the final dispersion at a concentration of between about 5 to about 99 percent (wt./wt.).

11. The process according to claim 4 wherein the liquid is selected from the group consisting of a hydrophobic liquid, a hydrophilic liquid, an aqueous liquid, or mixtures thereof.

12. The process according to claim4 wherein the concentration of the sol-gel precursors in the final dispersion is between about 5 to about 99 percent (wt./wt.).

13. The process according to claim 4 wherein the wetting agent is selected from the group consisting of a surfactant, a polymeric surfactant, or mixtures thereof.

14. The process according to claim 13 wherein the surfactant is selected from the group consisting of an anionic surfactant, a cationic surfactant, an amphoteric surfactant, a nonionic surfactant, or mixtures thereof.

15. The process according to any of the proceeding claims, wherein the oil is selected from a group consisting of mineral oil, dimethicone, cyclomethicone, alkyl siloxanes, alkylether siloxanes, dimethicone copolyols, C12-15 Alkyl Benzoate, Isostearyl Benzoate, PPG- 15 Stearyl Ether Benzoate, Octyldodecyl Benzoate, Stearyl Benzoate, Methyl Gluceth-20 Benzoate, Poloxamer 182 Dibenzoate, Poloxamer 105 Benzoate, Transcutol, Bernel Ester, Diethylhexylmaleate, Diethylhexylsebacate, Diethylhexyladipate, Diisopropyladipate, Diisopropylsebacate, Diisopropylmaleate, Ethylhexylsalicylate, Tridecylsalicylate, Butiloctylsahcylate, Isopropylmyristate, or mixtures thereof.
